# EUROPÄISCHE PATENTSCHRIFT

(11) **EP 1 358 906 B1**
(45) Veröffentlichungstag und Bekanntmachung des Hinweises auf die Patenterteilung: **25.03.2009**
(21) Anmeldenummer: 03090095.5
(22) Anmeldetag: 04.04.2003
(51) Int. Cl.: A61N 1/372

(54) **Anordnung zur Überwachung, Kalibrierung und Optimierung einer Steuerung eines elektromedizinischen Implantats**
Assembly for monitoring, calibrating and optimizing the controller of an electromedical implant
Assemblage de surveillance, d'étalonnage et d'optimisation de contrôleur d'un implant electromedical

(30) Priorität: 05.04.2002 DE 10216216
(43) Veröffentlichungstag der Anmeldung: 05.11.2003
(73) Patentinhaber: BIOTRONIK GmbH & Co. KG, 12359 Berlin (DE)
(72) Erfinder: Czygan, Gerald, 91054 Buckenhof (DE); Schaldach, Max, deceased (DE)
(74) Vertreter: Lindner-Vogt, Karin L.

(56) Entgegenhaltungen:
- EP-A- 0 148 486
- EP-B1- 0 215 730
- US-A- 5 318 593
- T. WETZIG: "Technische Anwendung myokardialer Summenaktionspotentiale als Informationsträger in der Herz-Kreislaufregelung" (Promotionsarbeit), Kapitel "5.7 Stellgliedsimulation in der Herz-Kreislaufregelung" 1997 , UNIVERSITÄT ERLANGEN-NÜRNBERG , ERLANGEN XP002249866 * Seite 62 - Seite 67 *
- WETZIG T ET AL: "ANALYSE EVOZIERTER MYOKARDPOTENTIALE IN ABHAENGIGKEIT VON ERGOMETRISCHER BELASTUNG UND SCHLAGFREQUENZ FUER DIE ANWENDUNG IN FREQUENZADAPTIVEN HERZSCHRITTMACHERN ANALYSIS OF THE VENTRICULAR EVOKED RESPONSE AS A FUNCTION OF PHYSICAL LOAD AND HEART RATE FOR USE IN RATE ADAPTIVE PACEMAKERS" BIOMEDIZINISCHE TECHNIK, FACHVERLAG SCHIELE UND SCHOEN GMBH. BERLIN, DE, Bd. 40, Nr. 1/2, 1995, Seiten 9-13, XP000487028 ISSN: 0013-5585

## Beschreibung

Die Erfindung betrifft eine Anordnung zur Überwachung und Einstellung einer Steuerung eines elektromedizinischen Implantates für eine intrakardiale Herztherapie mittels einer externen Steuer- und Auswerteeinheit mit den Merkmalen des Anspruchs 1.

Elektromedizinische Implantate, wie Herzschrittmacher und Defibrillatoren, haben sich als ein außerordentlich erfolgreiches Instrument für die Elektrotherapie brady- und tachykarder Rhythmusstörungen erwiesen. Die Aufgabe derartiger Implantate beschränkt sich dabei nicht nur auf die bloße Abgabe von elektrischen Impulsen zum Vorhof- oder Ventrikelmyokard, um dort eine Depolarisation auszulösen. Vielmehr sind in modernen Schrittmachern und Defibrillatoren Sensoren und Auswerteschaltungen vorgesehen, die eine gesteuerte oder geregelte Frequenzanpassung, Anpassung von Pulsamplitude, Pulsweite und AV-Zeit zur Therapieoptimierung erlauben. Auf diese Weise soll das kardiovaskuläre System des Patienten in seiner Reaktion auf physische oder psychische Belastung möglichst dem natürlichen Regelmechanismus nachgebildet werden (frequenz- oder ratenadaptive Herzschrittmacher).
Gegenwärtige elektromedizinische Implantate weisen zur Realisation dieses Zieles ein modulares Design auf. Ein solches Implantat umfasst in der Regel Elemente, wie eine zentrale Steuerlogik, eine Sensorik zur Erfassung von Körperfunktionen und zur intrakardialen Signalaufzeichnung im Myokard des rechten Vorhofes oder Ventikels, im Myokard verankerte Elektroden inklusive einer Schaltung zur Regelung der Impulsabgabe, eine Energiequelle für die Betriebsspannung und eine Takt- und Zeitgebereinheit. Die genannten einzelnen Komponenten sind selbstverständlich in ihrer Bauweise in einem hohen Maße variabel den jeweils vorliegenden Bedingungen anpassbar. Auf eine ausführliche Beschreibung der einzelnen Komponenten und ihrer sinnvollen Kombinationen soll daher, da bekannt, verzichtet werden.
Schon aus schaltungstechnischen Gründen hat es sich als sinnvoll erwiesen, die Funktionen des Implantates auf die Steuerung der Impulsabgabe und der intrakardialen Signalaufzeichnung zu beschränken. Weitergehende Aufgaben, wie beispielsweise die Überwachung und Optimierung der Steuerung selbst können in der Regel nur in einem sehr begrenzten Maße wahrgenommen werden. Dies liegt nicht nur daran, dass die Realisation derartig komplexer Steuerschaltungen auf dem begrenzten Bauraum des Implantats zur Zeit an ihre machbaren Grenzen stößt. Vielmehr würde eine solche komplexe Steuerschaltung auch zu einem deutlich erhöhten Energieverbrauch führen, sodass sich die Lebensdauer des Implantates verkürzen würde. Ebenso kann in der Regel eine fehlerhafte Steuerlogik nicht sich selbst überprüfen und neu einstellen. Aus diesen Gründen hat es sich als sinnvoll erwiesen, das elektromedizinische Implantat und auch einen externen Programmgeber mit je einem Sender und Empfänger auszustatten. Über die Telemetrieeinheiten der beiden Komponenten findet ein bidirektionaler Datenaustausch statt, der Stimulations- und Diagnostikparametern bzw. aufgezeichneten intrakardialen Signal- und Betriebsparametern umfasst.

Die US 4,705,043 von Imran zeigt beispielhaft einen solchen externen Programmgeber, der die Auswertung der übertragenen intrakardialen Signale übernimmt und dem Implantat Stimulationsparameter übermittelt. Nachteilig hieran ist, dass der externe elektrische Stimulator wesentlicher Teil der Steuerung des Implantates ist und bei seinem Ausfall das Implantat allenfalls in einer vorgegebenen Grundkonfiguration weiterarbeiten kann.

In der DE 37 22 829 C2 ist ein Verfahren beschrieben, bei dem ein implantierbares elektromedizinisches Gerät mit einem externen Programmgeber unter Übertragung codierter Signale in Kommunikation treten kann. Der externe Programmgeber tritt in der Regel temporär mit dem Implantat in Kontakt, wobei die die Impulsauslösung bestimmende Betriebsart und Parameter vom Programmgeber zu einem internen Programmierteil des Implantates übermittelt oder verändert werden. Der externe Programmgeber ist dabei so ausgelegt, dass er nicht eigenständig mit dem Implantat kommuniziert, sondern vielmehr erst nachdem ihn eine bestimmt Aufgabe durch den Programmierer (im allgemeinen dem Arzt) vorgegeben wird. Entsprechend vereinfacht sind solche Programmgeber in der Praxis in ihrem technischem Aufbau. Sie bestehen im wesentlichen aus der Sende- und Empfängereinheit und zahlreichen Schnittstellen, über die Peripheriegeräte angeschlossen werden können. Eine autarke Steuerung ist nicht vorgesehen. Dementsprechend muss der Patient bei der Programmierung, Optimierung und Durchführung von Belastungstest in der Klinik verweilen, wobei er zusätzlich durch eine umfangreiche Verkabelung behindert ist. Die ungewohnte Umgebung führt bekanntermaßen zu einem sogenannten Weißkitteleffekt, bei dem das kardiovaskuläre System des Patienten eben gerade nicht den Alltagsbedingungen unterliegt und damit eine Optimierung der Stimulation erschwert oder gar verhindert wird.

Aus T. WETZIG: "Technische Anwendung myokardialer Summenaktionspotentiale als Informationsträger in der Herz-Kreislaufregelung" (Promotionsarbeit), Kapitel "5.7 Stellgliedsimulation in der Herz-Kreislaufregelung" 1997 , UNIVERSITÄT ERLANGEN-NÜRNBERG , ERLANGEN, Seiten 62 bis 67; ist ein System bekannt, das einen festfrequenten Herzschrittmacher und einen in einem externen Gerät angeordneten P-Regler umfasst. Herzschrittmacher und externes Gerät stehen in telemetrischer Verbindung miteinander. Eine vom Herzschrittmacher erfasste ventrikuläre evozierte Reizantwort (VER) wird an das externe Gerät übertragen und dort analysiert, so dass der Regler im externen Gerät aus der ausgewerteten VER eine Stimulationsfrequenz bestimmt, die dann auf den Herzschrittmacher zurückübertragen wird.

Das gleiche System ist auch in WETZIG T ET AL: "ANALYSE EVO-ZIERTER MYOKARDPOTENTIALE IN ABHAENGIGKEIT VON ER-GOMETRISCHER BELASTUNG UND SCHLAGFREQUENZ FUER DIE ANWENDUNG IN FREQUENZADAPTIVEN HERZSCHRITTMA-CHERN ANALYSIS OF THE VENTRICULAR EVOKED RESPONSE AS A FUNCTION OF PHYSICAL LOAD AND HEART RATE FOR USE IN RATE ADAPTIVE PACEMAKERS" BIOMEDIZINISCHE TECHNIK, FACHVERLAG SCHIELE UND SCHOEN GMBH. BERLIN, DE, Bd. 40, Nr. 1/2, 1995, Seiten 9-13 beschrieben.

Aufgabe der vorliegenden Erfindung ist es daher, eine Anordnung zur Überwachung und Einstellung der Steuerung des elektromedizinischen Implantates für eine intrakardiale Herztherapie zur Verfügung zu stellen, mit der die Mobilität des Patienten wesentlich verbessert wird. Damit einhergehend soll insbesondere die Optimierung der Steuerparameter auf Grundlage von im Alltag aufgezeichneten intrakardialen Signalen verbessert werden. Das System soll möglichst eigenständig arbeiten und flexibel auf die gegebenenfalls veränderten Umstände reagieren können, ohne dass der Patient zeitaufwendige klinische Untersuchungen über sich ergehen lassen muss.
Diese Aufgabe wird durch die erfindungsgemäße Anordnung zur Überwachung und Einstellung einer Steuerung eines elektromedizinischen Implantates für eine intrakardiale Herztherapie mittels einer externen Steuer- und Auswerteeinheit mit den Merkmalen des Anspruchs 1 gelöst. Die Anordnung umfasst
(a) das elektromedizinische Implantat, welches folgende Bestandteile umfasst:
   - eine Takt- und Zeitgebereinheit zur Koordination eines zeitlichen Ablaufs von Steuervorgängen,
   - eine Energiequelle zur Bereitstellung einer Betriebsspannung und Stimulations- und Schockabgabe,
   - zumindest eine Elektrode, die mit dem Myokard des Herzens in Verbindung steht und zur Abgabe von elektrischen Impulsen geeignet ist,
   - eine Sensorik, die Messdaten über physiologische Größen im Bereich des Herzens und über interne Zustandsparameter des Implantats liefert,
   - eine Speichereinheit zur Hinterlegung von internen Steuersequenzen, Steuerparametern und Messdaten der Sensorik,
   - eine Telemetrieeinheit mit einem Sender und einem Empfänger zum bidirektionalen Datenaustausch mit der externen Steuer- und Auswerteeinheit und
   - eine zentrale, autark lauffähige Steuerlogik, die anhand der internen Steuersequenzen und Steuerparameter die Aktivitäten der zumindest einen Elektrode, der Telemetrieeinheit, der Sensorik und der Speichereinheit steuert, sowie
(b) die externe Steuer- und Auswerteeinheit gemäß Anspruchs 1.

Durch die autark läufige Steuerlogik des Implantates ist zunächst ein komfortabler Alltagsbetrieb sichergestellt, sodass das Implantat und die externe Steuer- und Auswerteeinheit nicht permanent in telemetrischem Kontakt stehen müssen. Die Steuer- und Auswerteeinheit mit ihrer autark lauffähigen Auswertelogik beinhaltet Sequenzen, die eine sehr viel umfangreichere Auswertung und Optimierung der Steuerung erlauben.

Erfindungsgemäß umfasst die Anordnung ein Expertensystem, welches alle Überwachungs- und Einstellungsaufgaben für die intrakardiale Herztherapie patientenindividuell bewertet und die den Überwachungs- und Einstellungsaufgaben zugrunde liegenden Steuersequenzen in interne Steuersequenzen für das Implantat und externe Steuersequenzen für die externe Auswerte- und Steuereinheit aufteilt. Ziel dieser Aufteilung ist es, das Implantat zwar für den Alltag weitgehend autark lauffähig zu machen, jedoch darüber hinaus gehende Aufgaben bei der Diagnostik und Therapie erst über die externe Auswerte- und Steuereinheit zu veranlassen. Sequenzen, die nach dem vorliegenden Patientenbild unnötig erscheinen oder nur der allgemeinen Überwachung des Betriebszustands des Implantats zugehörig sind, sollen nach Möglichkeit nicht im Implantat selbst hinterlegt werden. Vorzugsweise verbleiben im Implantat nur die internen Steuersequenzen, die zur Steuerung der Therapiefunktionen beziehungsweise Diagnostik bestimmter kardialer Ereignisse unbedingt notwendigen Funktion- und Diagnostikparameter umfassen. Dazu gehören vorzugsweise auch echtzeitabhängige Stimulations- und Diagnostiksequenzen. Insgesamt soll damit der Steueraufwand im Implantat reduziert werden, was letztendlich zu einer Energieeinsparung und damit Erhöhung der Lebensdauer führt.

Die Auswertelogik der externen Steuer- und Auswerteeinheit besitzt einen Speicher für eine komplexe Matrix, in die zumindest die übermittelten Daten des Implantats und weitere Steuergrößen einfließen. Mit Hilfe einer solchen Matrix lassen sich zügig die den Überwachungs- und Einstellungsaufgaben zugrunde liegenden Steuersequenzen und Steuerparameter ermitteln.

In einer bevorzugten Ausgestaltung des Erfindungsgedankens umfasst die Steuer- und Auswerteeinheit eine erste Eingabeeinheit zur Interaktion mit dem Patienten. Über diese Eingabeeinheit kann der Patient Steuergrößen eingeben, die Einfluss auf die Ermittlung der Steuersequenzen und -parameter in der Auswertelogik und damit indirekt auf die Steuerung des Implantats haben. Damit wird erstmalig eine aktivere Rolle des Patienten bei der Einstellung und Überprüfung der Diagnostik- und Stimulationsparameter zugelassen, die selbstverständlich durch entsprechende Programmierung auf bestimmte Bereiche / Aktion der Steuerlogik des Implantats begrenzt sein kann. Denkbar ist auch, dass die Eingabe über die erste Eingabeeinheit in verschiedenen Stufen erfolgen kann. Neben der direkten Eingriffsmöglichkeit in die Steuerung sollte eine reine Markerfunktion vorhanden sein. Der Patient kann z.B. durch Knopfdruck bestimmte Zustände oder Symptome lediglich dokumentieren bzw. bewerten.

Ferner ist bevorzugt, dass die externe Steuer- und Auswerteeinheit eine zweite Eingabeeinheit zum Datenaustausch mit weiteren Peripheriegeräten aufweist. Über diese zweite Eingabeeinheit können Steuersequenzen für die Auswertelogik aufgespielt bzw. verändert werden und/oder Daten aus der Speichereinheit für zusätzliche Auswertung ausgelesen werden. Umgekehrt können auf diesem Wege zeit- oder ereignisabhängige Steuergrößen der Matrix übermittelt werden. Hervorgehoben werden soll an dieser Stelle, dass für einen solchen Datenaustausch nicht mehr notwendigerweise der Patient vor Ort (in der Regel in der Klinik) vorstellig werden muss, sondern lediglich die externe Steuer- und Auswerteeinheit an die Peripheriegeräte angeschlossen werden muss. Der Datenaustausch kann dann insbesondere online über eine Telefonverbindung stattfinden.

Erfindungsgemäß werden die durch die Matrix ermittelten Steuersequenzen und Steuerparameter mit Gewichtungsfaktoren verknüpft. Anhand der Gewichtungsfaktoren wird ein zeitlicher Ablauf der einzelnen Überwachungs- und Einstellungsaufgaben in einem Interaktionsschema festgelegt. So kann beispielsweise ein Zeitintervall bis zur Einleitung der nächsten Routineüberprüfungen verkürzt werden, wenn die Auswertung der letzten Messungen signifikante Abweichungen vom medizinisch notwendigen Stimulationsverhalten erbracht hat. Zur zeitlichen Koordination des Datenaustausches weist die externe Steuer- und Auswerteeinheit eine Takt- und Zeitgebereinheit auf.

Die Gewichtungsfaktoren werden im Normalfall derart vorgegeben, dass die anstehende Überwachungs- und Einstellungsaufgaben vorzugsweise in einer Ruhephase des Patienten stattfinden. Auf diese Weise sollen Störungen im Alltagsablauf des Patienten infolge umfangreicher Änderungen der Stimulations- und Diagnostikparameter möglichst gering gehalten werden. Im Akutfall, beispielsweise wenn die Stimulationsparameter wesentlich von den medizinisch notwendigen Impulsabgaben abweichen, beginnt der Datenaustausch unmittelbar nach Erstellung des Interaktionsschemas. Natürlich kann der Optimierungsvorgang der Steuerung des Implantats online mit der externen Steuer- und Auswerteeinheit erfolgen, d.h. ein Datenaustausch kann Schlag auf Schlag stattfinden bis sich optimale Verhältnisse einstellen. Vorzugsweise wird auch eine Reihenfolge der während der Interaktion von der externen Steuer- und Auswerteeinheit auf das Implantat übermittelten Steuersequenzen und Steuerparameter durch die Gewichtungsfaktoren festgelegt. Damit kann beispielsweise die für den Patienten bedeutsamere Optimierung der Stimulationsparameter einer Routineüberprüfung des Implantates vorgezogen werden.

Eine in zuvor genannter Weise modifizierte externe Auswerte- und Steuereinheit wird gesondert beansprucht.

Weitere bevorzugte Ausgestaltungen der Erfindung ergeben sich aus den übrigen, in den Unteransprüchen genannten Merkmalen. Die Erfindung wird nachfolgend in einem Ausführungsbeispiel anhand von Zeichnungen näher erläutern. Es zeigen:
- Figur 1:: Eine Prinzipskizze zur Funktionsweise einer Anordnung zur Überwachung und Einstellung einer Steuerung eines elektromedizinischen Implantats für eine intrakardiale Herztherapie durch eine externe Steuer- und Auswerteeinheit und
- Figur 2:: ein Funktions-Blockschaltbild einer Auswertelogik der externen Steuer- und Auswerteeinheit.

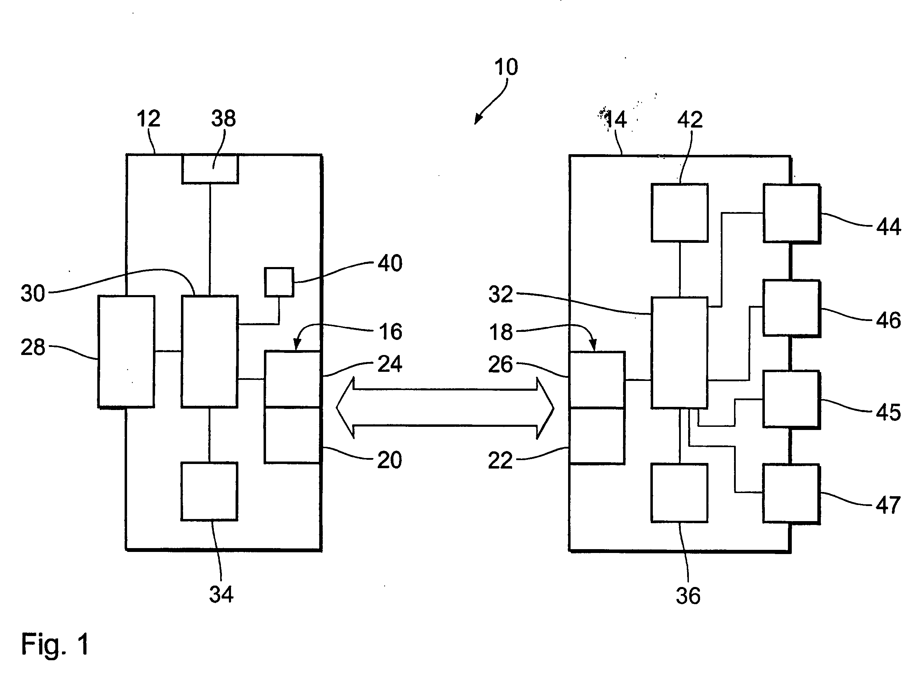

Die Figur 1 zeigt den prinzipiellen Aufbau einer Anordnung 10 zur Überwachung und Einstellung einer Steuerung eines elektromedizinischen Implantats 12 für eine intrakardiale Herztherapie durch eine externe Steuer und Auswerteeinheit 14. Das elektromedizinische Implantat 12 ist beispielsweise ein ratenadaptiver Herzschrittmacher.

Das Implantat 12 und die Steuer- und Auswerteeinheit 14 umfassen jeweils eine Telemetrieeinheit 16, 18 mit je einem Sender 20, 22 und Empfänger 24, 26, die zur bidirektionalen, telemetrischen Übertragung von Daten geeignet sind. Die auf telemetrischem Wege übertragenen Daten beinhalten beispielsweise Patienteninformationen wie das Implantationsdatum, das Datum der letzten Nachsorge, Angaben zur Symptomatik, etc. . Darüber hinaus können diagnostische Informationen, beispielsweise zur atrialen bzw. ventrikulären Diagnostik und Stimulation, übermittelt werden. Weiterhin werden Batterie- und Elektrodenparameter, insbesondere die aktuellen Werte von Batteriespannung, Batterieinnenwiderstand und Stromverbrauch weitergegeben, um eine realistische Abschätzung der zu erwartenden Betriebszeit zu ermöglichen. Des weiteren können über die Telemetrieeinheiten 16, 18 noch im folgenden näher erläuterte Messdaten einer Sensorik 28 und Steuersequenzen- und -parameter für eine Steuerlogik 30 des Implantats 12 bzw. Auswertelogik 32 der Auswerteund Steuereinheit 14 ausgetauscht werden. Aufbau und Funktionsweise derartiger Telemetrieeinheiten 16, 18 sind hinlänglich aus dem Stand der Technik bekannt und sollen daher an dieser Stelle nicht näher erläutert werden.

Das Implantat 12 umfasst ferner die Sensorik 28, mit der unmittelbar Parameter physikalischer oder physiologischer Natur erfasst werden können, die zur Steuerung oder Regelung der Therapie nutzbar sind. Beispiele hierfür sind Piezoschwinger oder kapazitive Sensoren zur Erfassung von Körperaktivität und Bewegungsenergie, Sensoren zur Erfassung einer zentralvenösen Bluttemperatur oder Sauerstoffsättigung, pH-Wert-Sensoren, Sensoren zur Erfassung einer Atemfrequenz, eines Atemminutenvolumens und Schlagvolumens des Herzens, Sensoren zur Bestimmung der Kontraktibilität bzw. Kontraktionsdynamik sowie Sensoren zur Erfassung von evozierten Myokardpotentialen. Auch hier wird auf eine ausführlichere Beschreibung der Sensorik 28 verzichtet, da diese bereits hinlänglich aus dem Stand der Technik bekannt ist.

Sowohl das Implantat 12, als auch die Steuer- und Auswerteeinheit 14 umfassen eine Speichereinheit 34, 36. Die Speichereinheit 34 des Implantats 12 dient zur Hinterlegung interner Steuersequenzen, Steuerparameter und Messdaten der Sensorik 28 und die Speichereinheit 36 der Steuer- und Auswerteeinheit 14 zur Hinterlegung von Steuersequenzen, Steuerparameter und übermittelter Daten.

Zur Stimulation und Diagnostik weist das Implantat 12 eine Elektrode 38 auf. Die Elektrode 38 steht mit dem Myokard des Herzens in Verbindung und ist zur Abgabe elektrischer Impulse geeignet. Grundsätzlich kann jede aus dem Stand der Technik bekannte Elektrode Einsatz finden, sofern sie die für die anstehenden Stimulations- und Diagnostikaufgaben verwendbar ist. Ferner weist die das Implantat 12 eine - hier nicht dargestellte - Energiequelle zur Bereitstellung der Betriebspannung auf (in der Regel eine Batterie).

Das Implantat 12 umfasst eine geeignete Schaltung, mit der sich die Steuerlogik 30 realisieren lässt, die die Aktivitäten aller vorhandenen Systemkomponenten bei der Stimulation und Diagnostik anhand interner Steuersequenzen und Steuerparameter koordiniert. Die Steuerlogik ist autark lauffähig, dass heißt sie kann für sich alleine alle die den Alltag betreffenden Stimulations- und Diagnostikaufgaben sicherstellen. Eine derartige zentrale Steuerlogik 30 ist üblicherweise eine integrierte Halbleiterschaltung, die mit anderen Systemkomponenten, insbesondere den Mitteln für die Telemetrie 16, 18, Sensorik 28, Speicherung 34, 36 und die Elektrodenansteuerung in Verbindung steht. Hier sei wiederum auf die im Stand der Technik zahlreichen beschriebenen Ausführungsformen verwiesen. Die der Ausführung der Erfindung dienenden Funktionskomponenten der Steuerlogik 30 sind in der Praxis teilweise softwaremäßig realisiert und untrennbar mit der übrigen Schrittmacherstruktur verflochten.

Für einen Herzschrittmacher, der zumindest eine Elektrode 38 zur Impulsanregung und Diagnostik im rechten Atrium und Ventrikel ausweist, lassen sie die einzelnen Aufgaben der Schrittmachersteuerlogik 30 in Form eines binären Automaten realisieren, der in Abhängigkeit von bestimmten Zeitintervallen und detektierten Signalen verschiedene Zustände annehmen kann. Die wichtigsten Zeitintervalle für den Steuerablauf beziehen sich beispielsweise auf Zeiträume die maximal zwischen zwei Eigenimpulsen der jeweiligen Kammern vergehen dürfen, ohne dass ein Stimulus ausgelöst wird. Ein AV-Delay wird bei modernen Schrittmachern einerseits nach detektierten Ereignissen verkürzt gegenüber stimulierten Ereignissen, um die Verzögerung zwischen Vorhof Stimulus und tatsächlicher Vorhofdepolarisation zu berücksichtigen (Latenzzeitkompensation). Zum anderen wird das AV-Delay zur Nachabbildung einer natürlichen Dromotrophie frequenzabhängig verkürzt, um auch bei höheren Herzraten eine optimale Synchronisation zu gewährleisten (dynamische AV-Zeit). Zu Einzelheiten bezügliche einer solchen Steuerlogik 30 wird auf das Buch von Schaldach, Electrotherapy of the Heart, Springer Verlag, Berlin, 1992 verwiesen. Es versteht sich von selbst, dass für die Realisation einer derartigen Steuerlogik 30 eine Takt- und Zeitgebereinheit 40 vorgesehen sein muss. Die Takt- und Zeitgebereinheit 40 ist beispielsweise ein Schwingquarz. Auch auf Seiten der externen Steuer- und Auswerteeinheit 14 ist ebenfalls eine Takt- und Zeitgebereinheit 42 zur zeitlichen Koordination der Steuervorgänge erforderlich.

Die externe Steuer- und Auswerteeinheit 14 beinhaltet weiterhin zwei Eingabeeinheiten 44, 46. Die erste Eingabeeinheit 44 soll eine Interaktion des Patienten ermöglichen. Auf diese Weise soll erreicht werden, dass der Patient in adäquater Weise auf möglicherweise auftretende physische Beschwerden reagieren und die Steuerung des Implantats 12 beeinflussen kann. Dabei kann in Abhängigkeit von den individuellen Fertigkeiten des Patienten ein Umfang der Eingriffsmöglichkeiten durch die Auswertelogik 32 festgelegt werden können. Ebenso ist denkbar, dass die Eingabe über die erste Eingabeeinheit 44 in verschiedenen Stufen erfolgen kann. Neben der direkten Eingriffsmöglichkeit in die Steuerung sollte eine reine Markerfunktion vorhanden sein. Der Patient kann z.B. durch Knopfdruck bestimmte Zustände oder Symptome lediglich dokumentieren bzw. bewerten.

Die zweite Eingabeeinheit 46 soll den Datenaustausch mit weiteren Peripheriegeräten ermöglich. Sollen beispielsweise umfangreiche statistische Auswertungen mit hohem Rechenaufwand durchgeführt werden oder in der Steuer- und Auswerteeinheit 14 zwischengespeicherte Daten einem Ausgabegerät zugeführt werden, so kann eine Kommunikation zu diesen Peripheriegeräten über die besagte zweite Eingabeeinheit 46 erfolgen. In umgekehrter Richtung lassen sich die Steuersequenzen und Steuerparameter der Auswertelogik 32 selbst neuen Erfordernissen anpassen und können gegebenenfalls völlig neu aufgespielt werden. Die zweite Eingabeeinheit 46 kann insbesondere ein Modem zum Anschluss an eine Telefonleitung umfassen, so dass eine Fernwartung des Auswerte- und Steuergerätes 14 möglich ist.

Eine weitere Eingabeeinheit 45 ist für den Anschluss externer Sensoren an die Steuer- und Auswerteeinheit 14 vorgesehen. Derartige Sensoren können insbesondere Lagesensoren, Beschleunigungssensoren, Sensoren zur Erfassung eines Oberflächen-EKG's oder Blutdrucksensoren sein. Die von den Sensoren erfassten Messdaten fließen als Steuergrößen in die Auswertelogik 32 ein. Denkbar ist auch, dass ein oder mehrere Sensoren bereits fest in die Steuerund Auswerteeinheit 14 integriert sind (Eingabeeinheit 47). In Vordergrund bei der Realisation steht jedoch Mobilität und einfache Handhabbarkeit der konkreten Steuer- und Auswerteeinheit 14.

Die externe Steuer- und Auswerteeinheit 14 umfasst ferner die autark lauffähige Auswertelogik 32, die in Abhängigkeit von den telemetrisch übermittelten Daten des Implantats 12 und noch näher erläuterten Steuergrößen die Steuerung des Implantats 12 überwacht und einstellt. Dazu besitzt die Steuer- und Auswerteeinheit 14 einen matrixartig organisierten Speicher 48. Der Speicher 48 kann in gängiger Halbleitertechnologie gefertigt oder zumindest in Teilen softwaremäßig realisiert werden. Ein solche Auswertelogik 32 umfasst zudem Funktionskomponenten wie eine Prozessoreinheit zur datentechnischen Verarbeitung, Arbeitsspeicher und weitere elektronische Elemente, die beispielsweise die Kommunikation mit den Eingabeeinheiten 44, 45, 46, 47 der Telemetrieeinheit 16 und der Sensorik 28 ermöglichen. In der Praxis sind die der Ausführung der Erfindung dienenden Funktionskomponenten teilweise softwaremäßig realisiert und untrennbar mit der übrigen Steuer- und Auswertestruktur verflochten. Derartige elektronische Systeme sind seit langem bekannt und variieren infolge des technologischen Fortschritts in Ihrer Auslegung erheblich, so dass auf eine nähergehende Beschreibung verzichtet wird. Zu Versorgung mit der notwendigen Betriebsspannung wird eine - hier nicht dargestellte - Energiequelle benötigt. Dazu dient beispielsweise ein wiederaufladbarer Akkumulator.

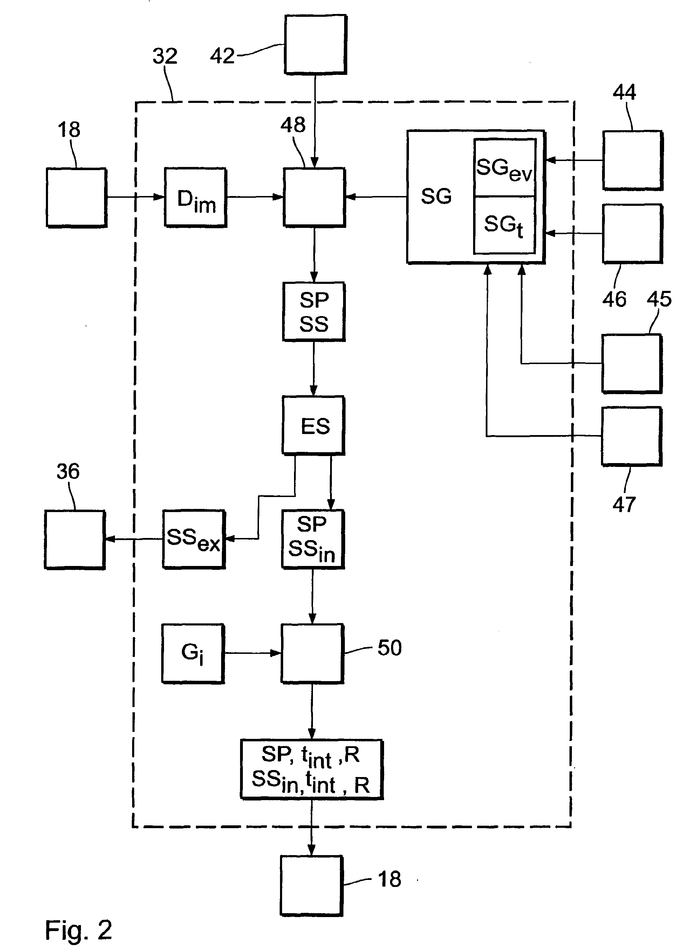

Die Figur 2 zeigt in schematischer Weise die Funktionsweise einer autark lauffähigen Auswertelogik 32 der Steuer- und Auswerteeinheit 14. Die Auswertelogik 32 verfügt dazu über die komplexe zentrale Matrix im Speicher 48, in die die übermittelten Daten Dᵢₘ des Implantats 12 und die Steuergrößen SG einfließen. Zur zeitlichen Koordination wir über die Takt- und Zeitgebereinheit ein Zeitsignal t ebenfalls eingespielt. Die Steuergrößen SG lassen sich systematisch in zeitabhängige Steuergrößen SGₜ und ereignisabhängige Steuergrößen SGₑᵥ gliedern.

Unter die zeitabhängigen Steuergrößen SGₜ fallen u.a. Parameter für Überwachungsaufgaben, die
- eine Routineüberprüfung der gesamten Steuerlogik 30 des Implantats 12 oder ihre Teilbereiche,
- eine Routineüberprüfung der gesamten Steuerlogik 30 des Implantats 12 oder ihrer Teilbereiche infolge einer vom Patienten getätigten Eingabe und
- eine Routineüberprüfung der gesamten Steuerlogik 30 des Implantats 12 und ihrer Teilbereiche infolge einer Vorgabe mittels geeigneter Peripheriegeräte
in vorgebbaren Zeitintervallen erzwingen. Derartige Steuerungsroutinen der Auswertelogik 32 sollen einen störungsfreien Betrieb des Implantats 12 über lange Zeiträume sicherstellen.

Unter bestimmten Umständen ist es sinnvoll ereignisabhängige Steuergrößen SGₑᵥ in die Matrix der Auswertelogik 32 mit einfließen zu lassen. Als ereignisabhängige Steuergrößen SGₑᵥ kommen dabei insbesondere Parameter für Überwachungs- und Einstellungsaufgaben in Frage, die
- über die erste Eingabeeinheit 44 durch den Patienten eingegeben werden,
- die über die zweite Eingabeeinheit 46 mittels geeigneter Peripheriegeräte vorgegeben werden oder
- die über die Eingabeeinheiten 45, 47 mittels geeigneter Sensoren ermittelt werden.

Die zeit- und ereignisabhängigen Steuergrößen SGₜ, SGₑᵥ fließen zusammengefasst als Steuergrößen SG in die Matrix ein. Sie werden zusammen mit den Daten Dᵢₘ des Implantats 12 für die Ermittlung der Steuersequenzen SS und Steuerparameter SP für anstehende Überwachungs- und Einstellungsaufgaben genutzt.

Unter Steuersequenz SS ist in diesem Sinne eine programmierte Befehlskette zu verstehen, deren Abarbeitung zur Vorgabe bestimmter Steuerparameter SP für vorhandene Stellglieder der Implantatsteuerung führt. Die Steuerparameter SP werden demnach vor Ort ermittelt. Wenn die Steuerparameter SP in der Auswerte- und Steuereinheit 14 bereits ermittelt wurden und telemetrisch auf das Implantat 12 übertragen werden, so kann die Übertragung direkt an die Stellglieder des Implantats 12 erfolgen. Auf diese Weise kann auf eine Hinterlegung der Steuersequenz SS im Implantat 12 verzichtet werden.

Die Auswertelogik 32 umfasst ein Expertensystem ES, welches alle Überwachungs- und Einstellungsaufgaben für die intrakardiale Herztherapie patientenindividuell bewertet. Das Expertensystem ES dient dazu die den Überwachungs- und Einstellungsaufgaben zugrunde liegenden Steuersequenzen SS in interne Steuersequenzen SSᵢₙ für das Implantat 12 und externe Steuersequenzen SSₑₓ für die externe Auswerte- und Steuereinheit 14 aufzuteilen. Damit soll zum eine Dauer der Datenübertragung durch Reduktion der auszutauschenden Datenmenge verkürzt werden. Des weiteren sollen nur die für die autarke Lauffähigkeit tatsächlich notwendigen Steuersequenzen SS in dem Implantat 12 hinterlegt werden. Dies spart Speicherplatz und vermindert den Energieverbrauch, denn diese Sequenzen werden nun in der Folge nur noch in der Auswerte- und Steuereinheit 14 durchgeführt. Die daraus resultierenden Steuerparameter werden anschließend telemetrisch übertragen.

Die internen Steuersequenzen SSᵢₙ umfassen die zur Steuerung der Stimulationsfunktion beziehungsweise Diagnostik bestimmter kardialer Ereignisse unbedingt notwendigen Stimulations- und Diagnostiksequenzen umfassen. Leidet der Patient beispielsweise an einer normotopen Tachykardie des rechten Atriums ohne sonstige Reizbildungsstörungen, so ist es nicht notwendig ventrikuläre Steuersequenzen vorzugeben. Erst wenn eine nähergehende Auswertung in der Auswerte- und Steuereinheit 14, die Notwendigkeit auch für eine ventrikuläre Herztherapie sinnvoll erscheinen lässt, werden die notwendigen Steuersequenzen SS übertragen. Steuersequenzen SS, die in jedem Fall auf dem Implantat 12 zu installieren sind, betreffen selbstverständlich echtzeitabhängige Stimulations- und Diagnostiksequenzen.

Die nach der Selektion durch das Expertensystem ES verbleibenden internen Steuersequenzen SSᵢₙ und Steuerparameter SP werden zusätzlich mit Gewichtungsfaktoren Gᵢ verknüpft. Anhand der Gewichtungsfaktoren Gᵢ soll einerseits ein Zeitintervall tᵢₙₜ, in dem die Überwachungs- und Einstellungsaufgaben stattfinden sollen, festgelegt werden. Andererseits ist es auch möglich eine Reihenfolge R der während der Interaktionen von der externen Steuer- und Auswerteeinheit 14 auf das Implantat 12 übermittelten internen Steuersequenzen SSᵢₙ und Steuerparameter SP für die anstehenden Überwachungs- und Einstellungsaufgaben zu bestimmen (Erstellung eines Interaktionsschemas 50). So ist es sinnvoll, eine Neueinstellung der Steuerlogik 30 vor deren Überwachung durchzuführen. Die Gewichtungsfaktoren Gᵢ werden im Normalfall so gewählt, dass das Zeitintervall tᵢₙₜ für die Überwachung und Einstellung in eine Ruhepause des Patienten fällt. Bei akuten Ereignissen, beispielsweise einer diagnostizierten aktiven ektopischen Reizbildungsstörung oder infolge einer Patienteneingabe, wird das Zeitintervall tᵢₙₜ so vorgegeben, dass es unmittelbar nach Erstellung des Interaktionsschemas 50 beginnt. Unter Umständen wird die Verbindung zwischen der externen Steuer- und Auswerteeinheit 14 und dem Implantat 12 solange aufrecht erhalten bis die Optimierung der Steuerung abgeschlossen ist. Der Datenaustausch kann dann beispielsweise Schlag auf Schlag stattfinden.

Das Interaktionsschema 50 beinhaltet die einzelnen Aufgaben, die infolge der Matrixauswertung für die Überwachung und Einstellung der Steuerlogik 30 des Implantats 12 abzuarbeiten sind. Für die anstehenden Aufgaben werden Reihenfolge R und die Zeitintervalle tᵢₙₜ festgelegt und, sobald telemetrischer Kontakt besteht, abgearbeitet. Zur Sicherstellung der komplexen Überwachungsund Einstellungsaufgaben kann vorgesehen sein, dass die externe Steuer- und Auswerteeinheit 14 über eine akustische Ausgabeeinheit verfügt. Ist der Zeitraum zwischen dem letzten telemetrischen Kontakt bereits eine vorgegebene Zeitspanne lang nicht mehr zustande gekommen oder stehen dringende Aufgaben im Interaktionsschema 50 an, so kann der Patient durch akustische Signale darauf aufmerksam gemacht werden, den Kontakt herzustellen.

Die voran geschilderte Vorgehensweise kann sowohl für die Überwachung und Einstellung der Stimulationsparameter, also auch für die Überwachung und Einstellung der Diagnostikparameter genutzt werden. So kann beispielweise nach Auswertung der vom Implantat 12 übertragenen Daten Dᵢₘ eine differenziertere Analyse der Herzaktivität notwendig sein, um mit der so gewonnenen Information die Stimulation noch weiter zu verbessern beziehungsweise deren medizinische Notwendigkeit zu überprüfen.

### Bezugszeichenliste

- 10: Anordnung
- 12: elektromedizinisches Implantat
- 14: externe Steuer- und Auswerteeinheit
- 16, 18: Telemetrieeinheit
- 20, 22: Sender
- 24, 26: Empfänger
- 28: Sensorik
- 30: Steuerlogik
- 32: Auswertelogik
- 34, 36: Speichereinheit
- 38: Elektrode
- 40, 42: Takt- und Zeitgeber
- 44, 45, 46, 47: Eingabeeinheiten
- 48: matrixartiger Speicher
- 50: Interaktionsschema
- Dᵢₘ: übermittelte Daten des Implantats 12
- Gᵢ: Gewichtungsfaktor
- R: Reihenfolge der Überwachungs- und Einstellungsaufgaben
- SG: Steuergröße
- SGₜ: zeitabhängige Steuergröße
- SGₑᵥ: ereignisabhängige Steuergröße
- SP: Steuerparameter
- SS: Steuersequenz
- SSᵢₙ: interne Steuersequenz
- tᵢₙₜ: Zeitintervall für die Überwachungs- und Einstellungsaufgabe

## Patentansprüche

1. Externe Auswerte- und Steuereinheit (14), die folgende Bestandteile umfasst:
- eine Telemetrieeinheit (18) mit einem Sender (22) und einem Empfänger (26) zum bidirektionalen Datenaustausch mit dem elektromedizinischen Implantat (12),
- eine Takt- und Zeitgebereinheit (42) zur Koordination eines zeitlichen Ablaufs von Steuervorgängen,
- eine Energiequelle zur Bereitstellung einer Betriebsspannung,
- eine Speichereinheit (36) zur Hinterlegung von Steuersequenzen (SS) und Steuerparameter (SP) und übermittelter Daten (Dim) des Implantats (12),
- eine zentrale, autark lauffähige Auswertelogik (32), die die Aktivitäten der Telemetrieeinheit (18) und der Speichereinheit (36) steuert, mit der Takt- und Zeitgebereinheit (42) in Verbindung steht und anhand der übermittelten Daten (Dim) Steuersequenzen und -parameter (SS, SP) für die Überwachung und Einstellung des Implantats (12) ermittelt sowie deren Übertragung auf das Implantat (12) koordiniert,
die ein Expertensystem (ES) umfasst, welches ausgebildet ist, alle Überwachungs- und Einstellungsaufgaben für die intrakardiale Herztherapie patientenindividuell zu bewerten und welches die den Überwachungs- und Einstellungsaufgaben zugrunde liegenden Steuersequenzen (SS) in interne Steuersequenzen (SSin) für das Implantat (12) und externe Steuersequenzen (SSex) für die externe Auswerte- und Steuereinheit (14) aufteilt
die einen Speicher (48) für eine komplexe Matrix besitzt, in die zumindest die übermittelten Daten (Dim) des Implantats (12) und die Steuergrößen (SG) einfließen und die die den Überwachungs- und Einstellungsaufgaben zugrunde liegenden Steuerparameter (SP) und Steuersequenzen (SS) liefert
und die derart ausgebildet ist, dass die für den telemetrischen Datenaustausch bereitstehenden Steuerparameter (SP) und/oder Steuersequenzen (SS) mit Gewichtungsfaktoren (Gi) verknüpft werden anhand derer ein zeitlicher Ablauf (tint) und/oder eine Reihenfolge (R) der Übertragung der einzelnen Überwachungs- und Einstellungsaufgaben in einem Interaktionsschema (50) festgelegt wird.

2. Anordnung (10) zur Überwachung und Einstellung einer Steuerung eines elektromedizinischen Implantats (12) für eine intrakardiale Herztherapie mittels einer externen Steuer- und Auswerteeinheit (14), die ein elektromedizinisches Implantat (12) und eine externe Steuer- und Auswerteeinheit (14) gemäß Anspruch 1 umfasst und bei der
das elektromedizinischelmplantat (12) folgende Bestandteile umfasst:
- eine Takt- und Zeitgebereinheit (40) zur Koordination eines zeitlichen Ablaufs von Steuervorgängen,
- eine Energiequelle zur Bereitstellung einer Betriebsspannung und Stimulations- und Schockabgabe,
- zumindest eine Elektrode (38), die mit dem Myokard des Herzens in Verbindung steht und zur Abgabe von elektrischen Impulsen geeignet ist,
- eine Sensorik (28), die Messdaten über physiologische Größen im Bereich des Herzens und über interne Zustandsparameter des Implantats (12) liefert,
- eine Speichereinheit (34) zur Hinterlegung von internen Steuersequenzen (SSin), Steuerparametern (SP) und Messdaten der Sensorik (28),
- eine Telemetrieeinheit (16) mit einem Sender (24) und einem Empfänger (20) zum bidirektionalen Datenaustausch mit der externen Steuer- und Auswerteeinheit (14) und
- eine zentrale, autark lauffähige Steuerlogik (30), die anhand der internen Steuersequenzen (SSin) und Steuerparameter (SP) die Aktivitäten der zumindest einen Elektrode (28), der Telemetrieeinheit (16), der Sensorik (28) und der Speichereinheit (34) steuert.

3. Anordnung nach Anspruch 2, **dadurch gekennzeichnet, dass** die internen Steuersequenzen (SSin) die zur Steuerung der Therapiefunktionen beziehungsweise Diagnostik bestimmter kardialer Ereignisse unbedingt notwendigen Therapie- und Diagnostiksequenzen umfassen.

4. Anordnung nach Anspruch 2, **dadurch gekennzeichnet, dass** die internen Steuersequenzen (SSin) echtzeitabhängige Stimulations- und Diagnostiksequenzen umfassen.

5. Anordnung nach einem oder mehreren der Ansprüche 2 bis 4, **dadurch gekennzeichnet, dass** die externe Steuer- und Auswerteeinheit (14) eine erste Eingabeeinheit (44) zur Interaktion mit dem Patienten umfasst, über die dieser Steuergrößen (SG) eingeben und Einfluss auf die Auswertelogik (32) nehmen kann oder Markierungen und Bewertungen bestimmter Zustände oder Symptome vornehmen kann.

6. Anordnung nach einem oder mehreren der Ansprüche 2 bis 5, **dadurch gekennzeichnet, dass** die externe Steuer- und Auswerteeinheit (14) eine zweite Eingabeeinheit (46) zum Datenaustausch mit weiteren Peripheriegeräten umfasst, über die Steuergrößen (SG) eingegeben, Steuersequenzen (SS) für die Auswertelogik (32) verändert oder neu aufgespielt und/oder Daten aus der Speichereinheit (36) für eine zusätzliche Auswertung ausgelesen werden können.

7. Anordnung nach einem oder mehreren der Ansprüche 2 bis 6, **dadurch gekennzeichnet, dass** die externe Steuer- und Auswerteeinheit (14) eine weitere Eingabeeinheit (45) zum Datenaustausch mit externen Sensoren umfasst, über die Steuergrößen (SG) eingegeben werden können.

8. Anordnung nach einem oder mehreren der Ansprüche 2 bis 7, **dadurch gekennzeichnet, dass** die externe Steuer- und Auswerteeinheit (14) eine weitere Eingabeeinheit (47) mit integrierten Sensoren umfasst, über die Steuergrößen (SG) eingegeben werden können.

## Claims

1. An external control and analyzer unit (14), comprising the following components:
- a telemetry unit (18) with a transmitter (22) and a receiver (26) for bidirectional data exchange with the electromedical implant (12),
- a clock and timer unit (42) for coordination of a chronological sequence of control operations,
- a power source for supplying a battery voltage,
- a memory unit (36) for storing control sequences (SS) and control parameters (SP) and transmitted data (Dᵢₘ) of the implant (12),
- an autarchically runnable central analyzer logic (32), controlling the activities of the telemetry unit (18) and the memory unit (36), connected to the clock and timer unit (42) and determining control sequences and control parameters (SS, SP) for the monitoring and adjustment of the implant (12) on the basis of the data transmitted (Dᵢₘ) and coordinating their transmission to the implant (12),
comprising an expert system (ES), which is designed to evaluate all the monitoring and adjusting tasks for intracardiac therapy on an individual patient basis, dividing the control sequences (SS) on which the monitoring and adjusting tasks are based into internal control sequences (SSᵢₙ) for the implant (12) and external control sequences (88ₑₓ) for the external control and analyzer unit (14),
having a memory (48) for a complex matrix into which at least the transmitted data (Dᵢₘ) of the implant (12) and the control variables (SG) are input and which supplies the control parameters (SP) and control sequences (SS) on which the monitoring and adjusting tasks are based,
and designed so that the control parameters (SP) and/or control sequences (SS) supplied for the telemetric data exchange are linked with weighting factors (Gᵢ) on the basis of which a chronological sequence (tᵢₙₜ) and/or a sequence (R) of the transmission of individual monitoring and adjusting tasks is defined in an interaction scheme (50).

2. The arrangement (10) for monitoring and adjusting a control of an electromedical implant (12) for an intracardiac therapy by means of an external control and analyzer unit (14), which comprises an electromedical implant (12) and an external control and analyzer unit (14) according to Claim 1, and in which
the electromedical implant (12) comprises the following components:
- a clock and timer unit (40) for coordination of a chronological sequence of control processes,
- a power source for supplying a battery voltage and delivering stimulation and shock pulses,
- at least one electrode (38), which is connected to the myocardium of the heart and is suitable for delivering electric pulses,
- sensor system (28) which supplies measured data about physiological variables in the area of the heart and about internal parameters of state of the implant (12),
- a memory unit (34) for storing internal control sequences (SSᵢₙ), control parameters (SP) and measured data from the sensor system (28),
- a telemetry unit (16) with a transmitter (24) and a receiver (20) for bidirectional data exchange with the external control and analyzer unit (14), and
- an autarchically runnable central control logic (30) that controls the activities of the at least one electrode (28), the telemetry unit (16), the sensor system (28) and the memory unit (34) on the basis of the internal control sequences (SSᵢₙ) and control parameters (SP).

3. The arrangement according to Claim 2,
**characterized in that**
the internal control sequences (SSᵢₙ) comprising therapeutic and diagnostic sequences that are absolutely necessary for control of the therapy functions and/or diagnosis of certain cardiac events.

4. The arrangement according to Claim 2,
**characterized in that**
the internal control sequences (SSᵢₙ) comprise real-time-dependent stimulation and diagnostic sequences.

5. The arrangement according to any one or more of Claims 2 through 4,
**characterized in that**
the external control and analyzer unit (14) comprises a first input unit (44) for interaction with the patient by which the patient can enter control variables (SG) and can have an influence on the analyzer logic (32) or perform marking and evaluation of certain conditions or symptoms.

6. The arrangement according to any one or more of Claims 2 through 5,
**characterized in that** the external control and analyzer unit (14) comprises a second input unit (46) for data exchange with additional peripheral devices by which control variables (SG) can be input, control sequences (SS) for the analyzer logic (32) can be modified or performed and/or data from the memory unit (36) can be read out for an additional analysis.

7. The arrangement according to any one or more of Claims 2 through 6,
**characterized in that** the external control and analyzer unit (14) comprises an additional input unit (45) for data exchange with external sensors by which the control variables (SG) can be input.

8. The arrangement according to any one or more of Claims 2 through 7,
**characterized in that** the external control and analyzer unit (14) comprises an additional input unit (47) with integrated sensors by which the control variables (SG) can be input.

## Revendications

1. Unité d'analyse et de commande (14) externe, qui comporte les composants suivants :
- une unité de télémétrie (18) avec un émetteur (22) et un récepteur (26) pour l'échange de données bidirectionnel avec l'implant (12) électromédical,
- une unité génératrice de rythme et de temps (42) pour la coordination d'un déroulement dans le temps d'opérations de commande,
- une source d'énergie pour la fourniture d'une tension de service,
- une unité de stockage (36) pour le dépôt de séquences de commande (SS) et de paramètres de commande (SP) et de données (Dim) transmises de l'implant (12),
- une logique d'analyse (32) centrale, utilisable de façon indépendante, qui commande les activités de l'unité de télémétrie (18) et de l'unité de stockage (36), est en liaison avec l'unité génératrice de rythme et de temps (42) et détermine à l'aide des données (Dim) transmises des séquences et des paramètres de commande (SS, SP) pour le contrôle et le réglage de l'implant (12) et coordonne leur transmission à l'implant (12),
qui comporte un système d'expert (ES) qui est conçu pour évaluer toutes les tâches de contrôle et de réglage pour la thérapie cardiaque intracardiaque de façon individuelle par patient et qui divise les séquences de commande (SS) à la base des tâches de contrôle et de réglage en séquences de commande internes (SSin) pour l'implant (12) et en séquences de commande externes (SSex) pour l'unité d'analyse et de commande (14) externe,
qui possède une mémoire (48) pour une matrice complexe, dans laquelle au moins les données transmises (Dim) de l'implant (12) et les grandeurs de commande (SG) sont intégrées et qui fournit les paramètres de commande (SP) et séquences de commande (SS) à la base des tâches de contrôle et de réglage
et qui est conçue de telle sorte que les paramètres de commande (SP) et/ou séquences de commande (SS) mis à disposition pour l'échange de données télémétrique sont associés à des facteurs de pondération (Gi), à l'aide desquels un déroulement dans le temps (tint) et/ou un ordre de succession (R) de la transmission des tâches de contrôle et de réglage individuelles est défini dans un schéma d'interaction (50).

2. Dispositif (10) pour le contrôle et le réglage d'une commande d'un implant (12) électromédical pour une thérapie cardiaque intracardiaque au moyen d'une unité de commande et d'analyse (14) externe, qui comporte un implant (12) électromédical et une unité de commande et d'analyse (14) externe selon la revendication 1 et sur lequel
l'implant (12) électromédical comporte les composants suivants :
- une unité génératrice de rythme et de temps (40) pour la coordination d'un déroulement dans le temps d'opérations de commande,
- une source d'énergie pour la fourniture d'une tension de service et la délivrance d'une stimulation et d'un choc,
- au moins une électrode (38), qui est en liaison avec le myocarde du coeur et est appropriée pour la délivrance d'impulsions électriques ;
- un capteur (28), qui fournit des données de mesure sur des grandeurs physiologiques dans la zone du coeur et sur des paramètres d'état internes de l'implant (12),
- une unité de stockage (34) pour le dépôt de séquences de commande internes (SSin), paramètres de commande (SP) et données de mesure du capteur (28),
- une unité de télémétrie (16) avec un émetteur (24) et un récepteur (20) pour l'échange de données bidirectionnel avec l'unité de commande et d'analyse externe (14), et
- une logique de commande (30) centrale, utilisable de façon indépendante, qui commande à l'aide des séquences de commande (SSin) et paramètres de commande (SP) internes les activités de la au moins une électrode (28), de l'unité de télémétrie (16), du capteur (28) et de l'unité de stockage (34).

3. Dispositif selon la revendication 2, **caractérisé en ce que** les séquences de commande internes (SSin) comportent les séquences de thérapie et de diagnostic impérativement nécessaires pour la commande des fonctions de thérapie et le diagnostic de certains événements cardiaques.

4. Dispositif selon la revendication 2, **caractérisé en ce que** les séquences de commande internes (SSin) comportent des séquences de stimulation et de diagnostic dépendants du temps réel.

5. Dispositif selon l'une quelconque ou plusieurs des revendications 2 à 4, **caractérisé en ce que** l'unité de commande et d'analyse externe (14) comporte une première unité d'entrée (44) pour l'interaction avec le patient, par laquelle celui-ci peut entrer des grandeurs de commande (SG) et exercer une influence sur la logique d'analyse (32) ou effectuer des marquages et évaluations de certains états ou symptômes.

6. Dispositif selon l'une quelconque ou plusieurs des revendications 2 à 5, **caractérisé en ce que** l'unité de commande et d'analyse externe (14) comporte une seconde unité d'entrée (46) pour l'échange de données avec d'autres appareils périphériques, par lesquels des grandeurs de commande (SG) peuvent être entrées, des séquences de commande (SS) pour la logique d'analyse (32) peuvent être modifiées ou remises en forme et/ou des données provenant de l'unité de stockage (36) peuvent être lues pour une analyse supplémentaire.

7. Dispositif selon l'une quelconque ou plusieurs des revendications 2 à 6, **caractérisé en ce que** l'unité de commande et d'analyse externe (14) comporte une autre unité d'entrée (45) pour l'échange de données avec des capteurs externes, par lesquels des grandeurs de commande (SG) peuvent être entrées.

8. Dispositif selon l'une quelconque ou plusieurs des revendications 2 à 7, **caractérisé en ce que** l'unité de commande et d'analyse externe (14) comporte une autre unité d'entrée (47) avec des capteurs intégrés, par lesquels des grandeurs de commande (SG) peuvent être entrées.
